# EUROPEAN PATENT APPLICATION

(11) **EP 2 617 423 A1**
(43) Date of publication of application: **24.07.2013**
(21) Application number: 13152163.5
(22) Date of filing: 19.10.2007
(51) Int. Cl.: A61K 31/52, A61P 13/12, A61P 43/00

(54) **Purine derivatives for the treatment of cystic diseases**

(30) Priority: 19.10.2006 US 852760 P
(62) Divisional of application: 07852872.6
(71) Applicant: GENZYME CORPORATION, Waltham, MA 02451 (US)
(72) Inventor: Beskrovnaya, Oxana, Boston, MA 02116 (US); Bukanov, Nikolay, Boston, MA 02116 (US)
(74) Representative: Jones Day

(57) **Abstract**

Provided herein are methods of treatment of a cystic disease by administering a compound of Formula I. In certain embodiments, the compound for use in the methods provided herein is roscovitine or an analog thereof.

## Description

### RELATED APPLICATION DATA

This application claims priority to U.S. provisional application Serial No. 60/852,760, entitled "METHODS AND COMPOSITIONS FOR TREATMENT OF CYSTIC DISEASES" to Bukanov *et al.,* filed October 19, 2006. The contents of the provisional application are incorporated by reference herein in its entirety.

### FIELD

Provided herein are methods for the treatment of cystic diseases. In certain embodiments, the methods involve administering a compound that is a cyclin dependent kinase (cdk) inhibitor. In one embodiment, the cdk inhibitor for use in the methods provided herein is roscovitine.

### BACKGROUND

Renal cysts occur in one third of people older than 50 years. While most are simple cysts, renal cystic disease has multiple etiologies. Broad categories of the cystic disease include the following:
Congenital - Congenital cystic dysplasia;
Genetic - Autosomal recessive polycystic kidney disease (ARPKD), autosomal dominant polycystic kidney disease (ADPKD), nephronophthisis-medullary cystic kidney disease complex (NMCD);
Acquired - Simple cysts, acquired cystic disease;
Cysts associated with systemic disease - Von Hippel-Lindau syndrome (VHLS), tuberous sclerosis (TS) and
Malignancy - Renal cell carcinoma (RCC).

The most common larger cysts are acquired cysts, simple cysts, and cysts with ADPKD. Smaller cysts are associated with ARPKD, NMCD, and medullary sponge kidney (MSK). In adults, renal angiomyolipomas and RCC also may demonstrate cystic lesions.

### Polycystic Kidney Disease (PKD)

Polycystic kidney disease (PKD) describes several conditions in which fluid-filled cysts form in the kidneys. Cysts generally develop in weak segments of the tubules that carry urine from the glomeruli, The cyst's growth displaces healthy kidney tissue. The kidneys expand to accommodate the cyst, which can weigh as much as 20 pounds. There are many forms of PKD, both inherited forms and noninherited.

Autosomal dominant PKD (ADPKD) is the most common, inherited form. Symptoms of ADPKD usually develop between the ages of 30 and 40, but they can begin earlier, even in childhood. About 90 percent of all PKD cases are autosomal dominant PKD. ADPKD results from mutation in the PKD1 gene that encodes polycystin-1 (85% of the cases) or PKD2 gene that encodes polycystin-1 (15% of the cases).

Autosomal recessive PKD (ARPKD) is a rare, inherited form. Symptoms of autasomal recessive PKD begin in the earliest months of life, even in the womb.

Acquired cystic kidney disease (ACKD) develops in association with long-term kidney problems, especially in patients who have kidney failure and who have been on dialysis for a long time. Therefore it tends to occur in later years of life. It is not an inherited form of PKD.

The renal cystic diseases include, but are not limited to renal cyctic diseases such as: acquired renal cystic disease (ARCD), dialysis-associated cystic disease, autosomal dominant polycystic kidney disease (ADPKD), autosomal recessive polycystic kidney disease (ARPKD), congenital multicystic kidney (CMK), multicystic dysplastic kidney, end-stage renal disease (ESRD), medullary sponge kidney, MSK, nephronophthisis-medullary cystic kidney disease complex (NMCD), nephronophthisis-uremic medullary cystic disease complex, juvenile nephronophthisis, medullary cystic disease, renal cell carcinoma (RCC), tuberous sclerosis (TS), von Hippel-Lindau syndrome (VHLS).

When PKD causes kidneys to fail, which usually happens after many years, the patient requires dialysis or kidney transplantation. About one-half of people with the major type of PKD progress to kidney failure. PKD can cause cysts in the liver and problems in other organs, such as the heart and blood vessels in the brain. These complications distinguish PKD from the usually harmless "simple" cysts that often form in the kidneys in later years of life.

In the United States, about 600,000 people, and world wide about 12.5 million people have PKD, and it is a leading cause of kidney failure. Three factors determine cyst classification: its cause (acquired, inherited), its features (complicated, simple, multiple, single), and its location (outer (cortical) or inner (medullary) kidney tissue).

At this time, PKD has no cure. The treatments for PKD include medicine and surgery to reduce pain, antibiotics to resolve infections, dialysis to replace functions of failed kidneys and kidney transplantation. Therefore, there is a need for developing more efficient treatments of PKD.

### SUMMARY

In one embodiment, provided herein are methods for treating, ameliorating or preventing multiple cystic diseases. The cystic diseases include, but are not limited to renal cyctic diseases such as: acquired renal cystic disease (ARCD), dialysis-associated cystic disease, autosomal dominant polycystic kidney disease (ADPKD), autosomal recessive polycystic kidney disease (ARPKD), congenital multicystic kidney (CMK), multicystic dysplastic kidney, end-stage renal disease (ESRD), medullary sponge kidney (MSK), nephronophthisis-medullary cystic kidney disease complex (NMCD), nephronophthisis-uremic medullary cystic disease complex, juvenile nephronophthisis, medullary cystic disease, renal cell carcinoma (RCC), tuberous sclerosis (TS), von Hippel-Lindau syndrome (VHLS). In one embodiment, provided herein are methods for treatment, amelioration or prevention of polycystic kidney disease.

The methods provided herein are effected by administering a compound of formula I or a pharmaceutically acceptable derivative thereof, 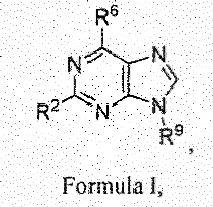 wherein the variables are selected such that the compound is effective in the treatment of a cystic disease. In certain embodiments, the methods encompass administering roscovitine or a pharmaceutically acceptable derivative thereof. In certain embodiments, the methods encompass administering R-roscovitine or a pharmaceutically acceptable derivative thereof.

Also provided are articles of manufacture containing packaging material, a compound of formula I or a pharmaceutically acceptable derivative thereof and a label that indicates that a compound of formula I or a pharmaceutically acceptable derivative thereof is used for treatment of a cystic disease.

### DETAILED DESCRIPTION

### A. DEFINITIONS

Unless defined otherwise, all technical and scientific terms used herein have the same meaning as is commonly understood by one of ordinary skill in the art. All patents, applications, published applications and other publications are incorporated by reference in their entirety. In the event that there are a plurality of definitions for a term herein, those in this section prevail unless stated otherwise.

As used herein, "cystic disease" refers to a disease selected from acquired renal cystic disease, ARCD, dialysis-associated cystic disease, autosomal dominant polycystic kidney disease, ADPKD, autosomal recessive polycystic kidney disease, ARPKD congenital multicystic kidney, CMK, multicystic dysplastic kidney, end-stage renal disease, ESRD, medullary sponge kidney, MSK, nephronophthisis-medullary cystic kidney disease complex, NMCD, nephronophthisis-uremic medullary cystic disease complex, juvenile nephronophthisis, medullary cystic disease, renal cell carcinoma, RCC, tuberous sclerosis, TS, and von Hippel-Lindau syndrome, VHLS.

As used herein, "polycystic kidney disease" or "PKD" refers to a condition characterized by the growth of numerous cysts in the kidneys. The cysts are filled with fluid. PKD cysts can slowly replace much of the mass of the kidneys, reducing kidney function and leading to kidney failure. The signs and symptoms of PKD include, but are not limited to pain in the back and lower sides, frequent urination, headaches, urinary tract infections, blood in the urine, cysts in the kidneys and other organs.

As used herein "roscovitine" refers to 6-benzylamino-2-(R)-[(1-ethyl-2-hydroxy)ethylamino]-9-isoprapylpurine. The chemical structure of roscovitine is described elsewhere herein.

As used herein "pulse dosing" refers to dosing of a drug that produces escalating drug levels early in the dosing interval followed by a prolonged drug-free period. For example, the pulse dosing schedules can involve a dosing interval of 4 weeks followed by 4 weeks of drug-free period, dosing interval of 3 weeks followed by 3 weeks of drug-free period, dosing interval of 2 weeks followed by 2 weeks of drug-free period, dosing interval of 1 weeks followed by 1 weeks of drug-free period, dosing interval of 5 days followed by 3 days of drug-free period. This type of drug delivery is therapeutically advantageous in certain embodiments because it requires reduced dose frequency and provides greater patient compliance. In certain embodiments, pulse dosing allows for a larger therapeutic window.

As used herein "drug-free period" refers to an interval in the pulse dosing schedule that does not involve administering a compound provided herein. The drug-free interval follows a period during which a compound is administered.

As used herein, pharmaceutically acceptable derivatives of a compound include salts, esters, enol ethers, enol esters, acetals, ketals, orthoesters, hemiacetals, hemiketals, acids, bases, solvates, hydrates or prodrugs thereof. Such derivatives may be readily prepared by those of skill in this art using known methods for such derivatization. The compounds produced may be administered to animals or humans without substantial toxic effects and either are pharmaceutically active or are prodrugs. Pharmaceutically acceptable salts include, but are not limited to, amine salts, such as but not limited to N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamineand other alkylamines, piperazine and tris(hydroxymethyl)aminomethane; alkali metal salts, such as but not limited to lithium, potassium and sodium; alkali earth metal salts, such as but not limited to barium, calcium and magnesium; transition metal salts, such as but not limited to zinc; and inorganic salts, such as but not limited to, sodium hydrogen phosphate and disodium phosphate; and also including, but not limited to, salts of mineral acids, such as but not limited to hydrochlorides and sulfates; and salts of organic acids, such as but not limited to acetates, lactates, malates, tartrates, citrates, ascorbates, succinates, butyrates, valerates, mesylates, and fumarates. Pharmaceutically acceptable esters include, but are not limited to, alkyl, alkenyl, alkynyl, aryl, aralkyl and cycloalkyl esters of acidic groups, including, but not limited to, carboxylic acids, phosphoric acids, phosphinic acids, sulfonic acids, sulfinic acids and boronic acids. Pharmaceutically acceptable enol ethers include, but are not limited to, derivatives of formula C=C(OR) where R is hydrogen, alkyl, alkenyl, alkynyl, aryl, aralkyl, cycloalkyl. Pharmaceutically acceptable enol esters include, but are not limited to, derivatives of formula C=C(OC(O)R) where R is hydrogen, alkyl, alkenyl, alkynyl, aryl, aralkyl, or cycloalkyl. Pharmaceutically acceptable solvates and hydrates are complexes of a compound with one or more solvent or water molecules, or 1 to about 100, or 1 to about 10, or one to about 2, 3 or 4, solvent or water molecules.

As used herein, EC₅₀ refers to a dosage, concentration or amount of a particular test compound that elicits a dose-dependent response at 50% of maximal expression of a particular response that is induced, provoked or potentiated by the particular test compound.

It is to be understood that the compounds provided herein may contain chiral centers. Such chiral centers may be of either the (R) or (S) configuration, or may be a mixture thereof. Thus, the compounds provided herein may be enantiomerically pure, or be stereoisomeric or diastereomeric mixtures. As such, one of skill in the art will recognize that administration of a compound in its (R) form is equivalent, for compounds that undergo epimerization *in vivo,* to administration of the compound in its (S) form.

As used herein, substantially pure means sufficiently homogeneous to appear free of readily detectable impurities as determined by standard methods of analysis, such as thin layer chromatography (TLC), gel electrophoresis, high performance liquid chromatography (HPLC) and mass spectrometry (MS), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers. In such instances, further purification might increase the specific activity of the compound. The instant disclosure is meant to include all such possible isomers, as well as, their racemic and optically pure forms. Optically active (+) and (-), (R)- and (S)-, or (D)- and (L)-isomers may be prepared using chiral synthons or chiral reagents, or resolved using conventional techniques, such as reverse phase HPLC. When the compounds described herein contain olefinic double bonds or other centers of geometric asymmetry, and unless specified otherwise, it is intended that the compounds include both E and Z geometric isomers. Likewise, all tautomeric forms are also intended to be included.

As used herein, the nomenclature alkyl, alkoxy, carbonyl, *etc*. is used as is generally understood by those of skill in this art.

As used herein, alkyl, alkenyl and alkynyl carbon chains, if not specified, contain from 1 to 20 carbons, or 1 to 16 carbons, and are straight or branched. Alkenyl carbon chains of from 2 to 20 carbons, in certain embodiments, contain 1 to 8 double bonds, and the alkenyl carbon chains of 2 to 16 carbons, in certain embodiments, contain 1 to 5 double bonds. Alkynyl carbon chains of from 2 to 20 carbons, in certain embodiments, contain 1 to 8 triple bonds, and the alkynyl carbon chains of 2 to 16 carbons, in certain embodiments, contain 1 to 5 triple bonds. Exemplary alkyl, alkenyl and alkynyl groups herein include, but are not limited to, methyl, ethyl, propyl, isopropyl, isobutyl, n-butyl, sec-butyl, tert-butyl, isopentyl, neopentyl, tert-pentyl, isohexyl, ethene, propene, butene, pentene, acetylene and hexyne. As used herein, lower alkyl, lower alkenyl, and lower alkynyl refer to carbon chains having from about 1 or about 2 carbons up to about 6 carbons. As used herein, "alk(en)(yn)yl" refers to an alkyl group containing at least one double bond and at least one triple bond.

As used herein, "cycloalkyl" refers to a saturated mono- or multicyclic ring system, in certain embodiments of 3 to 10 carbon atoms, in other embodiments of 3 to 6 carbon atoms; cycloalkenyl and cycloalkynyl refer to mono- or multicyclic ring systems that respectively include at least one double bond and at least one triple bond. Cycloalkenyl and cycloalkynyl groups may, in certain embodiments, contain 3 to 10 carbon atoms, with cycloalkenyl groups, in further embodiments, containing 4 to 7 carbon atoms and cycloalkynyl groups, in further embodiments, containing 8 to 10 carbon atoms. The ring systems of the cycloalkyl, cycloalkenyl and cycloalkynyl groups may be composed of one ring or two or more rings which may be joined together in a fused, bridged or spiro-connected fashion. "Cycloalk(en)(yn)yl" refers to a cycloalkyl group containing at least one double bond and at least one triple bond.

As used herein, "substituted alkyl," "substituted alkenyl," "substituted alkynyl," "substituted cycloalkyl," "substituted cycloalkenyl," and "substitued cycloalkynyl" refer to alkyl, alkenyl, alkynyl, cycloalkyl, cycloalkenyl and cycloalkynyl groups, respectively, that are substituted with one or more substituents, in certain embodiments one to three or four substituents, where the substituents are as defined herein, generally selected from Q.

As used herein, "aryl" refers to aromatic monocyclic or multicyclic groups containing from 6 to 19 carbon atoms. Aryl groups include, but are not limited to groups such as fluorenyl, substituted fluorenyl, phenyl, substituted phenyl, naphthyl and substituted naphthyl.

As used herein, "heteroaryl" refers to a monocyclic or multicyclic aromatic ring system, in certain embodiments, of about 5 to about 15 members where one or more, in one embodiment 1 to 3, of the atoms in the ring system is a heteroatom, that is, an element other than carbon, including but not limited to, nitrogen, oxygen or sulfur. The heteroaryl group may be optionally fused to a benzene ring. Heteroaryl groups include, but are not limited to, furyl, imidazolyl, pyrrolidinyl, pyrimidinyl, tetrazolyl, thienyl, pyridyl, pyrrolyl, N-methylpyrrolyl, quinolinyl and isoquinolinyl.

As used herein, a "heteroarylium" group is a heteroaryl group that is positively charged on one or more of the heteroatoms.

As used herein, "heterocyclyl" refers to a monocyclic or multicyclic non-aromatic ring system, in one embodiment of 3 to 10 members, in another embodiment of 4 to 7 members, in a further embodiment of 5 to 6 members, where one or more, in certain embodiments, 1 to 3, of the atoms in the ring system is a heteroatom, that is, an element other than carbon, including but not limited to, nitrogen, oxygen or sulfur. In embodiments where the heteroatom(s) is(are) nitrogen, the nitrogen is optionally substituted with alkyl, alkenyl, alkynyl, aryl, heteroaryl, aralkyl, heteroaralkyl, cycloalkyl, heterocyclyl, cycloalkylatkyl, heterocyclylalkyl, acyl, guanidino, or the nitrogen may be quaternized to form an ammonium group where the substituents are selected as above.

As used herein, "substituted aryl," "substituted heteroaryl" and "substituted heterocyclyl" refer to aryl, heteroaryl and heterocyclyl groups, respectively, that are substituted with one or more substituents, in certain embodiments one to three or four substituents, where the substituents are as defined herein, generally selected from Q.

As used herein, "aralkyl" refers to an alkyl group in which one of the hydrogen atoms of the alkyl is replaced by an aryl group.

As used herein, "heteroaralkyl" refers to an alkyl group in which one of the hydrogen atoms of the alkyl is replaced by a heteroaryl group.

As used herein, "halo", "halogen" or "halide" refers to F, Cl, Br or I.

As used herein, pseudohalides or pseudohalo groups are groups that behave substantially similar to halides. Such compounds can be used in the same manner and treated in the same manner as halides. Pseudohalides include, but are not limited to, cyano, thiocyanate, selenocyanate, trifluoromethoxy, and azide.

As used herein, "haloalkyl" refers to an alkyl group in which one or more of the hydrogen atoms are replaced by halogen. Such groups include, but are not limited to, chloromethyl, trifluoromethyl and 1-chloro-2-fluoroethyl.

As used herein, "alkynyl" refers to a straight, branched or cyclic, in certain embodiments straight or branched, divalent aliphatic hydrocarbon group, in one embodiment having from 1 to about 20 carbon atoms, in another embodiment having from 1 to 12 carbons. In a further embodiment alkynyl includes lower alkynyl. There may be optionally inserted along the alkynyl group one or more oxygen, sulfur, including S(=O) and S(=O)₂ groups, or substituted or unsubstituted nitrogen atoms, including -NR- and -N⁺RR- groups, where the nitrogen substituent(s) is(are) alkyl, aryl, aralkyl, heteroaryl, heteroaralkyl or COR', where R' is alkyl, aryl, aralkyl, heteroaryl, heteroaralkyl, -OY or NYY', where Y and Y' are each independently hydrogen, alkyl, aryl, heteroaryl, cycloalkyl or heterocyclyl. Alkynyl groups include, but are not limited to, methylene (-CH₂-), ethylene (-CH₂CH₂-), propylene (-(CH₂)₃-), methylenedioxy (-O-CH₂-O-) and ethylenedioxy (-O-(CH₂)₂-O-), The term "lower alkynyl" refers to alkynyl groups having 1 to 6 carbons. In certain embodiments, alkynyl groups are lower alkynyl, including alkynyl of 1 to 3 carbon atoms.

As used herein, "alkenylene" refers to a straight, branched or cyclic, in one embodiment straight or branched, divalent aliphatic hydrocarbon group, in certain embodiments having from 2 to about 20 carbon atoms and at least one double bond, in other embodiments 1 to 12 carbons. In further embodiments, alkenylene groups include lower alkenylene. There may be optionally inserted along the alkenylene group one or more oxygen, sulfur or substituted or unsubstituted nitrogen atoms, where the nitrogen substituent is alkyl. Alkenylene groups include, but are not limited to, —CH=CH— CH=CH— and —CH=CH—CH₂—. The term "lower alkenylene" refers to alkenylene groups having 2 to 6 carbons. In certain embodiments, alkenylene groups are lower alkenylene, including alkenylene of 3 to 4 carbon atoms.

As used herein, "alkynylene" refers to a straight, branched or cyclic, in certain embodiments straight or branched, divalent aliphatic hydrocarbon group, in one embodiment having from 2 to about 20 carbon atoms and at least one triple bond, in another embodiment 1 to 12 carbons. In a further embodiment, alkynylene includes lower alkynylene. There may be optionally inserted along the alkynylene group one or more oxygen, sulfur or substituted or unsubstituted nitrogen atoms, where the nitrogen substituent is alkyl. Alkynylene groups include, but are not limited to, -C≡C-C≡C-, -C≡C- and -C≡C-CH₂-. The term "lower alkynylene" refers to alkynylene groups having 2 to 6 carbons. In certain embodiments, alkynylene groups are lower alkynylene, including alkynylene of 3 to 4 carbon atoms.

As used herein, "cycloalkynyl" refers to a divalent saturated mono- or multicyclic ring system, in certain embodiments of 3 to 10 carbon atoms, in other embodiments 3 to 6 carbon atoms; cycloalkenylene and cycloalkynylene refer to divalent mono- or multicyclic ring systems that respectively include at least one double bond and at least one triple bond. Cycloalkenylene and cycloalkynylene groups may, in certain embodiments, contain 3 to 10 carbon atoms, with cycloalkenylene groups in certain embodiments containing 4 to 7 carbon atoms and cycloalkynylene groups in certain embodiments containing 8 to 10 carbon atoms. The ring systems of the cycloalkynyl, cycloalkenylene and cycloalkynylene groups may be composed of one ring or two or more rings which may be joined together in a fused, bridged or spiro-connected fashion. "Cycloalk(en)(yn)ylene" refers to a cycloalkynyl group containing at least one double bond and at least one triple bond.

As used herein, "substituted alkynyl," "substituted alkenylene," "substituted alkynylene," "substituted cycloalkynyl," "substituted cycloalkenylene," and "substitued cycloalkynylene" refer to alkynyl, alkenylene, alkynylene, cycloalkynyl, cycloalkenylene and cycloalkynylene groups, respectively, that are substituted with one or more substituents, in certain embodiments one to three or four substituents, where the substituents are as defined herein, generally selected from Q.

As used herein, "arylene" refers to a monocyclic or polycyclic, in certain embodiments monocyclic, divalent aromatic group, in one embodiment having from 5 to about 20 carbon atoms and at least one aromatic ring, in another embodiment 5 to 12 carbons. In further embodiments, arylene includes lower arylene. Arylene groups include, but are not limited to, 1,2-, 1,3- and 1,4-phenylene. The term "lower arylene" refers to arylene groups having 5 or 6 carbons.

As used herein, "heteroarylene" refers to a divalent monocyclic or multicyclic aromatic ring system, in one embodiment of about 5 to about 15 members where one or more, in certain embodiments 1 to 3, of the atoms in the ring system is a heteroatom, that is, an element other than carbon, including but not limited to, nitrogen, oxygen or sulfur.

As used herein, "heterocyclylene" refers to a divalent monocyclic or multicyclic non-aromatic ring system, in certain embodiments of 3 to 10 members, in one embodiment 4 to 7 members, in another embodiment 5 to 6 members, where one or more, including 1 to 3, of the atoms in the ring system is a heteroatom, that is, an element other than carbon, including but not limited to, nitrogen, oxygen or sulfur.

As used herein, "substituted arylene," "substituted heteroarylene" and "substituted heterocyclylene" refer to arylene, heteroarylene and heterocyclylene groups, respectively, that are substituted with one or more substituents, in certain embodiments one to three of four substituents, where the substituents are as defined herein, generally selected from Q.

Where the number of any given substituent is not specified (e.g., "hydroxyalkyl"), there may be one or more substituents present. For example, " hydroxyalkyl " may include one or more hydroxyl groups.

As used herein "subject" is an animal, such as a mammal, including human, such as a patient.

As used herein, and unless otherwise specified, the terms "treat," "treating" and "treatment" contemplate an action that occurs while a patient is suffering from the specified disease or disorder, which reduces the severity of the disease or disorder, or retards or slows the progression of the disease or disorder. Treatment also encompasses any pharmaceutical use of the compositions herein, such as use for treating PKD.

As used herein, amelioration of the symptoms of a particular disorder by administration of a particular pharmaceutical composition refers to any lessening of the symptoms, whether permanent or temporary, lasting or transient that can be attributed to or associated with administration of the composition.

As used herein, unless otherwise specified, the terms "prevent," "preventing" and "prevention" contemplate an action that occurs before a patient begins to suffer from the specified disease or disorder, which inhibits or reduces the severity of the disease or disorder.

As used herein, and unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

As used herein, and unless otherwise specified, the terms "therapeutically effective amount" and "effective amount" of a compound mean an amount sufficient to provide a therapeutic benefit in the treatment, prevent and/or management of a disease, to delay or minimize one or more symptoms associated with the disease or disorder to be treated. The terms "therapeutically effective amount" and "effective amount" can encompass an amount that improves overall therapy, reduces or avoids symptoms or causes of disease or disorder, or enhances the therapeutic efficacy of another therapeutic agent.

As used herein, and unless otherwise specified, the term "prophylactically effective amount" of a compound means an amount sufficient to prevent a disease or disorder, or one or more symptoms associated with the disease or disorder, or prevent its recurrence. The term "prophylactically effective amount" can encompass an amount that improves overall prophylaxis or enhances the prophylactic efficacy of another prophylactic agent.

The terms "co-administration" and "in combination with" include the administration of two therapeutic agents either simultaneously, concurrently or sequentially with no specific time limits. In one embodiment, both agents are present in the cell or in the patient's body at the same time or exert their biological or therapeutic effect at the same time. In one embodiment, the two therapeutic agents are in the same composition or unit dosage form. In another embodiment, the two therapeutic agents are in separate compositions or unit dosage forms.

### B. METHODS OF TREATMENT

Provided herein are methods for treatment, amelioration or prevention of a cystic disease by administering a cdk-2 inhibitor of Formula I 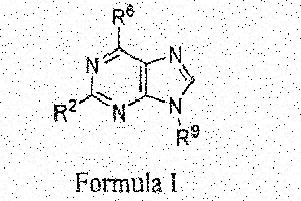 or a pharmaceutically acceptable derivative thereof, wherein
R² and R⁶ are each independently selected from halo, pseudohalo, alkyl, alkenyl, alkynyl, aryl, heteroaryl, cycloalkyl, heterocyclyl, R-NH-, R-NH-NH-, NH₂-R¹-NH- and R-NH-R'-NH-;
R⁹ is hydrogen, alkyl, alkenyl, alkynyl, aryl, heteroaryl, cycloalkyl, heterocyclyl, R-NH-, R-NH-NH-, NH₂-R¹-NH- or R-NH-R¹-NH-;
R is H, alkyl, alkenyl, alkynyl, aryl, heteroaryl, cycloalkyl or a heterocyclyl;
R' is alkylene, alkenylene, alkynylene, cycloalkynyl, heterocyclylene, arylene, or heteroarylene;
R, R', R², R⁶, and R⁹ groups are optionally substituted with one or more Q groups, in certain embodiments, one, two, three or four Q groups selected from -OH, - COOH, halo, pseudohalo, amino, alkyl, alkenyl, alkynyl, cycloalkyl, heterocyclyl, aryl and heteroaryl groups.

In one embodiment, the compounds are of Formula I or pharmaceutically acceptable derivatives thereof, wherein
R² and R⁶ are each independently selected from halo, pseudohalo, alkyl, alkenyl, alkynyl, aryl, heteroaryl, cycloalkyl, heterocyclyl, R-NH-, R-NH-NH-, NH₂-R¹-NH- and R-NH-R'-NH-;
R⁹ is hydrogen, alkyl, alkenyl, alkynyl, aryl, heteroaryl, cycloalkyl, heterocyclyl, R-NH-, R-NH-NH-, NH₂-R¹-NH- or R-NH-R¹-NH-;
R is H, alkyl, alkenyl, alkynyl, aryl, heteroaryl, cycloalkyl or a heterocyclyl;
R¹ is alkylene, alkenylene, alkynylene, cycloalkynyl, heterocyclylene, arylene, or heteroarylene;
R, R¹, R², R⁶, and R⁹ groups are optionally substituted with one or more Q groups, in certain embodiments, one, two, three or four Q groups selected from -OH, halo, pseudohalo, amino, alkyl, alkenyl, alkynyl, cycloalkyl, heterocyclyl, aryl and heteroaryl groups. The compounds of Formula I have activity as cdk inhibitors, in certain embodiments, as cdk-2 inhibitors. Certain of the compounds are disclosed in U.S. Patent No. 5,316,456, which is incorporated herein by reference.

In certain embodiments, R is alkyl, aralkyl, hydroxyalkyl, aryl, cycloalkyl or heterocyclyl. In certain embodiments, R¹ is a substituted or unsubstituted alkynyl, arylene or cycloalkynyl group.

In certain embodiments, Q is -OH, -COOH, halo, pseudohalo, amino or alkyl.

In certain embodiments, Q is -OH, halo, pseudohalo, amino or alkyl.

In one embodiment, k² is hydroxyalkylamino. In one embodiment, R² is (1-ethyl-2-hydroxy)ethylamino. In one embodiment, R⁶ is aralkylamino. In another embodiment, R⁶ is benzylamino. In one embodiment, R⁹ is lower alkyl. In another embodiment, R⁹ is isopropyl.

In certain embodiments, R², R⁶ and R⁹ are as indicated in the following table 1: **TABLE 1**

| R² | R⁶ | R⁹ |
|---|---|---|
| 3-hydroxypropylamino | benzylamino | isopropyl |
| 2-hydroxypropylamino | benzylamino | isopropyl |
| t-D,L-hydroxymethylpropylamino | benzylamino | isopropyl |
| aminoethylamino | benzylamino | isopropyl |
| 2-hydroxypropylamino | isopentenyl | isopropyl |
| 2-hydroxypropylamino | cyclohexylmethylamino | methyl |
| chloro | isopentenylamino | isopropyl |
| (2R)-2-hydroxymethylpyrrolidin-1-yl | benzylamino | isopropyl-(9H) |
| N-benzylaminoethanol | benzylamino | isopropyl-(9H) |
| (R,S)-amino-hexanol | benzylamino | isopropyl-(9H) |
| (S)-amino-2-phenylethanol | benzylamino | isopropyl-(9H) |
| (R)-amino-2-phenylethanol | benzylamino | isopropyl-(9H) |
| (R)-amino-3-phenylethanol | benzylamino | isopropyl-(9H) |
| (R,S)-amino-pentanol | benzylamino | isopropyl-(9H) |
| (R)-amino-propanol | benzylamino | isopropyl-(9H) |
| (S)-amino-propanol | benzylamino | isopropyl-(9H) |
| (R)-N-pyrrolidinemethanol | (3-iodo)benzylamino | isopropyl-(9H) |
| (R)-N-pyrrolidinemethanol | benzylamino (9H) | cyclopentyl- |

In certain embodiments, the compound for use in the methods provided herein is selected from 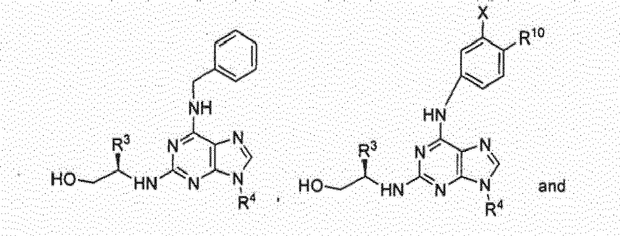 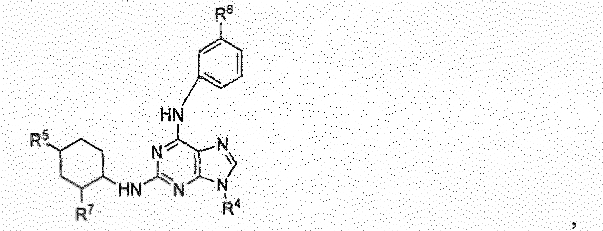 wherein R³ and R⁴ are each independently H, methyl, ethyl or isopropyl; R¹⁰ is H or COOH; R⁸ is H, halo or pseudohalo; R⁵ is H or OH; R⁷ is H or NH₂ and X is halo.

In certain embodiments, the compound for use in the methods provided herein is selected from 2-(2-hydroxyethylamino)-6-benzylamino-9-methylpurine, known as olomoucine, 2-(2'-Hydroxyethylamino)-6-benzy)amino-9-isopropylpurine, also called N⁹-isopropylolomoucine, (2*R*)-2-[[6-[(3-chlorophenyl)amino]-9-propan-2-ylpurin-2-yl]amino]-3-methylbutan-1-ol, also called purvalanol A and (2*R*)-2-[[6-((3-chloro-4-carboxyphenyl)amino]-9-(1-methylethyl)-9*H*-purin-2-yl]amino]-3-methyl-1-butanol, also called purvalanol B. In certain embodiments, the compound for use in the methods provided herein is

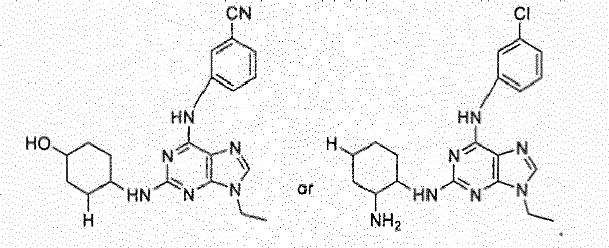

In certain embodiments, the compound for use in the methods provided herein is selected from 2-(1-D,L-hydroxymethylpropylamino)-6-benzylamino-9-isopropylpurine, non-crystalline 6-benzylamino-2-[(2R)-2-hydroxymethyl-pyrrolidin-1-yl]-9-isopropyl-(9H)-purine, 2-(R)-[6-benzylamino-9-isopropyl-(9H)-purin-2-yl]-amino-2-phenylethanol, 2-(R,S)-[6-benzylamino-9-isopropyl-(9H)-purin-2-yl]-amino-pentanol, 2-(R)-(6-benzylamino-9-isopropyl-(9H)-purin-2-yl]-amino-propanol, 2-(S)-[6-benzylamino-9-isopropyl-(9H)-purin-2-yl]-amino-propanol, 2-(R)-(-)-[6-(3-iodo)-benzylamino-9-isopropyl-(9H)-purin-2-yl]-N-pyrrolidine-methanol and 2-(R)-(-)-[6-benzylamino-9-cyclopentyl-(9H)-purin-2-yl]-N-pyrrolidine-methanol.

In certain embodiments, the compound for use in the methods provided herein is roscovitine. In other embodiments, the compound for use in the methods provided herein is R-roscovitine.

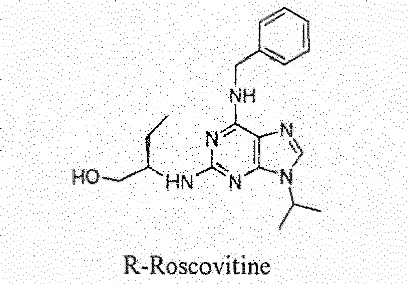

In one embodiment, the cystic disease is selected from acquired renal cystic disease (ARCD), dialysis-associated cystic disease, autosomal dominant polycystic kidney disease (ADPKD), autosomal recessive polycystic kidney disease (ARPKD), congenital multicystic kidney (CMK), multicystic dysplastic kidney, end-stage renal disease (ESRD), medullary sponge kidney, MSK, nephronophthisis-medullary cystic kidney disease complex (NMCD), nephronophthisis-uremic medullary cystic disease complex, juvenile nephronophthisis, medullary cystic disease, renal cell carcinoma (RCC), tuberous sclerosis (TS) and von Hippel-Lindau syndrome (VHLS).

In one embodiment, the cystic disease is a polycystic kidney disease (PKD). In certain embodiments, the signs and symptoms of polycystic kidney disease include, but are not limited to pain in the back and lower sides, headaches, frequent urination, urinary tract infections, blood in the urine, cysts in the kidneys and other organs. The diagnosis of PKD is done by methods known to one of skill in the art including, but not limited to ultrasound imaging of kidney cysts, ultrasound imaging of cysts in other organs and family medical history (genetic testing).

In certain embodiments, the methods provided herein encompass administration of roscovitine in pulse dosing. The schedule for pulse dosing can be determined empirically. In certain embodiments, the pulse dosing schedule is such that the drug is administered for a period of three consecutive weeks followed by a drug free interval of three weeks. In other embodiment, the drug is administered for a period of two consecutive weeks followed by a drug free interval of two weeks or the drug is administered for a period of one week followed by a drug free interval of one week.

### C. METHODS OF PREPARATION

The compounds provided herein can be prepared by methods known in the art. For example, see, U.S. Patent No. 6,316,456, which is incorporated herein by reference. An exemplary reaction scheme for preparation of roscovitine from 6-benzylamino-2-chloropurine is described below. 6-Benzylamino-2-chloropurine can be prepared as described by Hocart in Phytochemistry 1991, 30, 2477-2486.

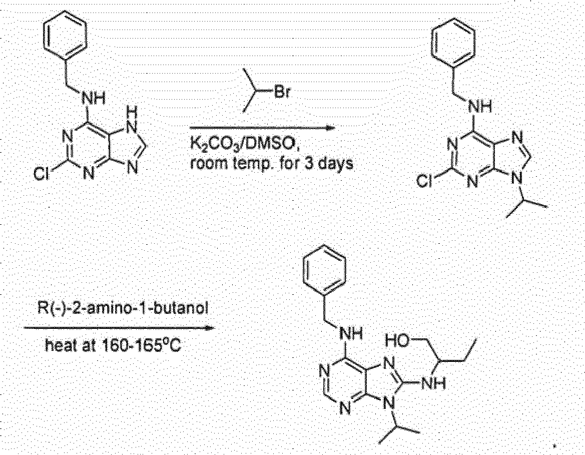

The product can be purified by column chromatography with MeOH/CHCl3 and crystallized in ethyl acetate.

### D. PHARMACEUTICAL COMPOSITIONS AND DOSAGE FORMS

Pharmaceutical compositions and dosage forms for use in the methods provided herein contain a compound provided herein in a pharmaceutically acceptable carrier and in amounts that are useful in the methods of treatment, amelioration or prevention of cystic diseases. In one embodiment, such methods include treatment, prevention or amelioration of a palycystic kidney disease.

The compounds provided for use herein are formulated into suitable pharmaceutical preparations such as solutions, suspensions, tablets, dispersible tablets, pills, capsules, powders, sustained release formulations or elixirs, for oral administration or in sterile solutions or suspensions for parenteral administration, as well as transdermal patch preparation and dry powder inhalers. In one embodiment, the formulations are prepared using techniques and procedures well known in the art (see, *e.g*., Ansel Introduction to Pharmaceutical Dosage Forms, Seventh Edition 1999).

In the compositions, effective concentrations of a compound provided herein is (are) mixed with a suitable pharmaceutical carrier or vehicle. The concentration of the compound provided herein in the compositions are effective for delivery of an amount, upon administration, that treats, prevents, or ameliorates one or more of the symptoms of conditions associated with a cystic disease.

In some embodiments, the compositions are formulated for single dosage administration. To formulate a composition, the weight fraction of a compound provided herein is dissolved, suspended, dispersed or otherwise mixed in a selected vehicle at an effective concentration such that the treated condition is relieved or ameliorated. Pharmaceutical carriers or vehicles suitable for administration of the compounds provided herein include any such carriers known to those skilled in the art to be suitable for the particular mode of administration.

In addition, a compound provided herein may be formulated as a sole pharmaceutically active ingredient in the composition or may be combined with other active ingredients. Liposomal suspensions, including tissue-targeted liposomes, may also be suitable as pharmaceutically acceptable carriers. These may be prepared according to methods known to those skilled in the art. For example, liposome formulations may be prepared as described in U.S. Pat. Nos. 4,522,811; 5,571,534. Briefly, liposomes such as multilamellar vesicles (MLV's) may be formed by drying down egg phosphatidyl choline and brain phosphatidyl serine (7:3 molar ratio) on the inside of a flask. A solution of a compound provided herein in phosphate buffered saline lacking divalent cations (PBS) is added and the flask shaken until the lipid film is dispersed. The resulting vesicles are washed to remove unencapsulated compound, pelleted by centrifugation, and then resuspended in PBS.

A compound provided herein is included in the pharmaceutically acceptable carrier in an amount sufficient to exert desired effect in the patient treated. The therapeutically effective concentration may be determined empirically by testing a compound provided herein in *in vitro* and *in vivo* systems known to one of skill in the art and then extrapolated therefrom for dosages for humans.

The concentration of a compound provided herein in the pharmaceutical composition will depend on absorption, inactivation and excretion rates of a compound provided herein, the dosage schedule, and amount administered as well as other factors known to those of skill in the art.

The composition, shape, and type of dosage forms provided herein will vary depending on their use. For example, a dosage form used in the acute treatment of a disease may contain larger amounts of one or more of the active ingredients it contains than a dosage form used in the chronic treatment of the same disease. Similarly, a parenteral dosage form may contain smaller amounts of one or more of the active ingredients it contains than an oral dosage form used to treat the same disease. These and other ways in which specific dosage forms provided herein will vary from one another will be readily apparent to those skilled in the art. *See, e.g.,* Remington's Pharmaceutical Sciences, 20th ed., Mack Publishing, Easton PA (2000).

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the compositions provided herein.

Thus, effective concentrations or amount of a compound provided herein is mixed with a suitable pharmaceutical carrier or vehicle for systemic, topical or local administration to form the pharmaceutical composition. A compound provided herein is included in an amount effective for treating, ameliorating or preventing a cystic disease.

The compositions are intended to be administered by a suitable route, including orally, parenterally, rectally, topically and locally. A compound provided herein is formulated and administered in unit-dosage forms such as tablets, capsules, pills, powders, granules, sterile parenteral solutions or suspensions, and oral solutions or suspensions, and oil-water emulsions containing suitable quantities of the active ingredient or multiple-dosage forms. Unit-dose forms as used herein refers to physically discrete units suitable for human and animal subjects and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampules and syringes and individually packaged tablets or capsules. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pints or gallons. Hence, multiple dose form is a multiple of unit-doses which are not segregated in packaging.

Lactose-free compositions provided herein can contain excipients that are well known in the art and are listed, for example, in the U.S. Pharmacopeia (USP) 25-NF20 (2002). In general, lactose-free compositions contains active ingredients, a binder/filler, and a lubricant in pharmaceutically compatible and pharmaceutically acceptable amounts. Particular lactose-free dosage forms contain active ingredients, microcrystalline cellulose, pre-gelatinized starch, and magnesium stearate.

Further provided are anhydrous pharmaceutical compositions and dosage forms comprising active ingredients, since water can facilitate the degradation of some compounds. For example, the addition of water (*e.g*., 5%) is widely accepted in the pharmaceutical arts as a means of simulating long-term storage in order to determine characteristics such as shelf-life or the stability of formulations over time. *See, e.g.,* Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms provided herein can be prepared using anhydrous or low moisture containing ingredients and low moisture or low humidity conditions.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (*e.g*., vials), blister packs, and strip packs.

### a. Compositions for Oral Administration

Oral pharmaceutical dosage forms are either solid, gel or liquid. The solid dosage forms are tablets, capsules, granules, and bulk powders. Types of oral tablets include compressed, chewable lozenges and tablets which may be enteric-coated, sugar-coated or film-coated. Capsules may be hard or soft gelatin capsules, while granules and powders may be provided in non-effervescent or effervescent form with the combination of other ingredients known to those skilled in the art. Such dosage forms contain predetermined amounts of active ingredients, and may be prepared by methods of pharmacy well known to those skilled in the art. *See generally,* Remington's Pharmaceutical Sciences, 20th ed., Mack Publishing, Easton PA (2000).

In certain embodiments, the formulations are solid dosage forms, preferably capsules or tablets. The tablets, pills, capsules, troches and the like can contain any of the following ingredients, or compounds of a similar nature: a binder, a filler, a diluent; a disintegrating agent, a lubricant, a glidant, a sweetening agent, and a flavoring agent. Examples of excipients that can be used in oral dosage forms provided herein include, but are not limited to, binders, fillers, disintegrants, and lubricants. Binders suitable for use in pharmaceutical compositions and dosage forms include, but are not limited to, com starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g*., Nos. 2208,2906,2910), microcrystalline cellulose, and mixtures thereof.

Suitable forms of microcrystalline cellulose include, but are not limited to, the materials sold as AVICEL-PH-101, AVICEL-PH-103, AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103 and Starch 1500 LM.

Examples of fillers suitable for use in the pharmaceutical compositions and dosage forms disclosed herein include, but are not limited to, talc, calcium carbonate (*e.g*., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions herein is present in from about 50 to about 99 weight percent of the pharmaceutical composition or dosage form.

Disintegrants are used in the compositions provided herein to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms provided herein. The amount of disintegrant used varies based upon the type of formulation, and is readily discernible to those of ordinary skill in the art. In some embodiment, the pharmaceutical compositions contain from about 0.5 to about 15 weight percent of disintegrant, preferably from about 1 to about 5 weight percent of disintegrant.

Disintegrants that can be used in pharmaceutical compositions and dosage forms provided herein include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms provided herein include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g*., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL®200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are used in an amount of less than about 1 weight percent of the pharmaceutical compositions or dosage forms into which they are incorporated.

If oral administration is desired, a compound provided herein could be provided in a composition that is formulated as enteric coating tablets, sugar-coated tablets, film-coated tablets or multiple compressed tablets. Enteric coating tablets protect the active ingredient from the acidic environment of the stomach. Sugar-coated tablets are compressed tablets to which different layers of pharmaceutically acceptable substances are applied. Film-coated tablets are compressed tablets which have been coated with a polymer or other suitable coating. Multiple compressed tablets are compressed tablets made by more than one compression cycle utilizing the pharmaceutically acceptable substances previously mentioned. Coloring agents may also be used in the above dosage forms. Flavoring and sweetening agents are used in compressed tablets, sugar-coated, multiple compressed and chewable tablets. Flavoring and sweetening agents are especially useful in the formation of chewable tablets and lozenges. The composition may also be formulated in combination with an antacid or other such ingredient.

When the dosage unit form is a capsule, it can contain, in addition to material of the above type, a liquid carrier such as a fatty oil. In a gelatin capsule, the solution or suspension containing a compound provided herein, in for example propylene carbonate, vegetable oils or triglycerides, is preferably encapsulated in the capsule. Such solutions, and the preparation and encapsulation thereof, are disclosed in U.S. Pat. Nos. 4,328,245; 4,409,239; and 4,410,545.

The active ingredient can also be mixed with other active materials which do not impair the desired action, or with materials that supplement the desired action, such as antacids, H2 blockers, and diuretics. Higher concentrations, up to about 98% by weight of the active ingredient may be included.

Liquid oral dosage forms include aqueous solutions, emulsions, suspensions, solutions and/or suspensions reconstituted from non-effervescent granules and effervescent preparations reconstituted from effervescent granules. Aqueous solutions include, for example, elixirs and syrups. Elixirs are clear, sweetened, hydroalcoholic preparations. Pharmaceutically acceptable carriers used in elixirs include solvents. Syrups are concentrated aqueous solutions of a sugar, for example, sucrose, and may contain a preservative.

An emulsion is a two-phase system in which one liquid is dispersed in the form of small globules throughout another liquid. Pharmaceutically acceptable carriers used in emulsions are non-aqueous liquids, emulsifying agents and preservatives. Suspensions use pharmaceutically acceptable suspending agents and preservatives. Pharmaceutically acceptable substances used in non-effervescent granules, to be reconstituted into a liquid oral dosage form, include diluents, sweeteners and wetting agents. Pharmaceutically acceptable substances used in effervescent granules, to be reconstituted into a liquid oral dosage form, include organic acids and a source of carbon dioxide. Coloring and flavoring agents are used in all of the above dosage forms.

Solvents include glycerin, sorbitol, ethyl alcohol and syrup. Examples of preservatives include glycerin, methyl and propylparaben, benzoic add, sodium benzoate and alcohol. Examples of non-aqueous liquids utilized in emulsions include mineral oil and cottonseed oil. Examples of emulsifying agents include gelatin, acacia, tragacanth, bentonite, and surfactants such as polyoxyethylene sorbitan monooleate. Suspending agents include sodium carboxymethylcellulose, pectin, tragacanth, Veegum and acacia.

Diluents include lactose and sucrose. Sweetening agents include sucrose, syrups, glycerin and artificial sweetening agents such as saccharin. Wetting agents include propylene glycol monostearate, sorbitan monooleate, diethylene glycol monolaurate and polyoxyethylene lauryl ether. Organic adds include citric and tartaric acid. Sources of carbon dioxide include sodium bicarbonate and sodium carbonate. Coloring agents include any of the approved certified water soluble FD and C dyes, and mixtures thereof. Flavoring agents include natural flavors extracted from plants such fruits, and synthetic blends of compounds which produce a pleasant taste sensation.

The pharmaceutical compositions containing active ingredients in micellar form can be prepared as described in U.S. Patent No. 6,354458. Such pharmaceutical compositions are particularly effective in oral, nasal and buccal applications.

In certain embodiments, formulations include, but are not limited to, those containing a compound provided herein, a dialkylated mono- or poly-alkynyl glycol, including, but not limited to, 1,2-dimethoxymethane, diglyme, triglyme, tetraglyme, polyethylene glycol-350-dimethyl ether, polyethylene glycol-550-dimethyl ether, polyethylene glycol-750-dimethyl ether wherein 350, 550 and 750 refer to the approximate average molecular weight of the polyethylene glycol, and one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, thiodipropionic acid and its esters, and dithiocarbamates.

Other formulations include, but are not limited to, aqueous alcoholic solutions including a pharmaceutically acceptable acetal. Alcohols used in these formulations are any pharmaceutically acceptable water-miscible solvents having one or more hydroxyl groups, including, but not limited to, propylene glycol and ethanol. Acetals include, but are not limited to, di(lower alkyl) acetals of lower alkyl aldehydes such as acetaldehyde diethyl acetal.

### b. Sustained Release Dosage Form

Active ingredients provided herein can be administered by sustained release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566, each of which is incorporated herein by reference. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable sustained -release formulations known to those of ordinary skill in the art, including those described herein, can be readily selected for use with the active ingredients provided herein.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g*., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

In certain embodiments, the agent may be administered using intravenous infusion, an implantable osmotic pump, a transdermal patch, liposomes, or other modes of administration. In one embodiment, a pump may be used *(see,* Sefton, CRC Crit. Ref Biomed. Eng. 14:201 (1987); Buchwald et al., Surgery 88:507 (1980); Saudek et al., N. Engl. J Med. 321:574 (1989). In another embodiment, polymeric materials can be used. In yet another embodiment, a controlled release system can be placed in proximity of the therapeutic target, *i.e*., thus requiring only a fraction of the systemic dose (*see, e.g.,* Goodson, Medical Applications of Controlled Release, vol. 2, pp. 115-138 (1984).

Preferably, a controlled release device is introduced into a subject in proximity of the site of inappropriate immune activation or a tumor. Other controlled release systems are discussed in the review by Langer (Science 249:1527-1533 (1990). The active ingredient can be dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylenevinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylenelvinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient contained in such parenteral compositions is highly dependent on the specific nature thereof, as well as the needs of the subject.

### c. Parenteral administration

Parenteral administration, generally characterized by injection, either subcutaneously, intramuscularly or intravenously is also contemplated herein. Injectables can be prepared in conventional forms, either as liquid solutions or suspensions, solid forms suitable for solution or suspension in liquid prior to injection, or as emulsions. Suitable excipients are, for example, water, saline, dextrose, glycerol or ethanol. In addition, if desired, the pharmaceutical compositions to be administered may also contain minor amounts of non-toxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins.

Parenteral administration of the compositions includes intravenous, subcutaneous and intramuscular administrations. Preparations for parenteral administration include sterile solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

If administered intravenously, suitable carriers include physiological saline or phosphate buffered saline (PBS), and solutions containing thickening and solubilizing agents, such as glucose, polyethylene glycol, and polypropylene glycol and mixtures thereof.

Pharmaceutically acceptable carriers used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Examples of aqueous vehicles include Sodium Chloride Injection, Ringers Injection, Isotonic Dextrose Injection, Sterile Water Injection, Dextrose and Lactated Ringers Injection. Nonaqueous parenteral vehicles include fixed oils of vegetable origin, cottonseed oil, corn oil, sesame oil and peanut oil. Antimicrobial agents in bacteriostatic or fungistatic concentrations must be added to parenteral preparations packaged in multiple-dose containers which include phenols or cresols, mercurials, benzyl alcohol, chlorobutanol, methyl and propyl p-hydroxybenzoic acid esters, thimerosal, benzalkonium chloride and benzethonium chloride. Isotonic agents include sodium chloride and dextrose. Buffers include phosphate and citrate. Antioxidants include sodium bisulfate. Local anesthetics include procaine hydrochloride. Suspending and dispersing agents include sodium carboxymethylcelluose, hydroxypropyl methylcellulose and polyvinylpyrrolidone. Emulsifying agents include Polysorbate 80 (TWEEN® 80). A sequestering or chelating agent of metal ions include EDTA. Pharmaceutical carriers also include ethyl alcohol, polyethylene glycol and propylene glycol for water miscible vehicles and sodium hydroxide, hydrochloric acid, citric acid or lactic acid for pH adjustment.

The concentration of a compound provided herein is adjusted so that an injection provides an effective amount to produce the desired pharmacological effect. The exact dose depends on the age, weight and condition of the patient or animal as is known in the art.

The unit-dose parenteral preparations are packaged in an ampule, a vial or a syringe with a needle. All preparations for parenteral administration must be sterile, as is known and practiced in the art.

Illustratively, intravenous or intraarterial infusion of a sterile aqueous solution containing an active ingredient is an effective mode of administration. Another embodiment is a sterile aqueous or oily solution or suspension containing an active material injected as necessary to produce the desired pharmacological effect.

Injectables are designed for local and systemic administration. In one embodiments, a therapeutically effective dosage is formulated to contain a concentration of at least about 0.1% w/w up to about 90% w/w or more, preferably more than 1% w/w of a compound provided herein to the treated tissue(s). The active ingredient may be administered at once, or may be divided into a number of smaller doses to be administered at intervals of time. It is understood that the precise dosage and duration of treatment is a function of the tissue being treated and may be determined empirically using known testing protocols or by extrapolation from in vivo or in vitro test data. It is to be noted that concentrations and dosage values may also vary with the age of the individual treated. It is to be further understood that for any particular subject, specific dosage regimens should be adjusted over time according to the individual need and the professional judgment of the person administering or supervising the administration of the formulations, and that the concentration ranges set forth herein are exemplary only and are not intended to limit the scope or practice of the claimed formulations.

A compound provided herein may be suspended in micronized or other suitable form or may be derivatized to produce a more soluble active product or to produce a prodrug. The form of the resulting mixture depends upon a number of factors, including the intended mode of administration and the solubility of a compound provided herein in the selected carrier or vehicle. The effective concentration is sufficient for ameliorating the symptoms of the condition and may be empirically determined.

### d. Lyophilized Powders

Of interest herein are also lyophilized powders, which can be reconstituted for administration as solutions, emulsions and other mixtures. They may also be reconstituted and formulated as solids or gels.

The sterile, lyophilized powder is prepared by dissolving the active ingredient, or a pharmaceutically acceptable derivative thereof, in a suitable solvent. The solvent may contain an excipient which improves the stability or other pharmacological component of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at about neutral pH. Subsequent sterile filtration of the solution followed by lyophilization under standard conditions known to those of skill in the art provides the desired formulation. Generally, the resulting solution will be apportioned into vials for lyophilization. Each vial will contain a single dosage (10-500 mg, preferably 100-300 mg) or multiple dosages of a compound provided herein. The lyophilized powder can be stored under appropriate conditions, such as at about 4°C to room temperature.

Reconstitution of this lyophilized powder with water for injection provides a formulation for use in parenteral administration. For reconstitution, about 1-50 mg, preferably 5-35 mg, more preferably about 9-30 mg of lyophilized powder, is added per mL of sterile water or other suitable carrier. The precise amount depends upon the selected compound. Such amount can be empirically determined.

### e. Topical Administration

Topical mixtures are prepared as described for the local and systemic administration. The resulting mixture may be a solution, suspension, emulsions or the like and are formulated as creams, gels, ointments, emulsions, solutions, elixirs, lotions, suspensions, tinctures, pastes, foams, aerosols, irrigations, sprays, suppositories, bandages, dermal patches or any other formulations suitable for topical administration.

The compounds provided herein may be formulated for local or topical application, such as for topical application to the skin and mucous membranes, in the form of gels, creams, and lotions. Topical administration is contemplated for transdermal delivery and also for administration mucosa, or for inhalation therapies.

### f. Compositions for Other Routes of Administration

Other routes of administration, such as topical application, transdermal patches, and rectal administration are also contemplated herein. For example, pharmaceutical dosage forms for rectal administration are rectal suppositories, capsules and tablets for systemic effect. Rectal suppositories are used herein mean solid bodies for insertion into the rectum which melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glycerin-gelatin, carbowax (polyoxyethylene glycol) and appropriate mixtures of mono-, di- and triglycerides of fatty acids. Combinations of the various bases may be used. Agents to raise the melting point of suppositories include spermaceti and wax. Rectal suppositories may be prepared either by the compressed method or by molding. In certain embodiments, the weight of a rectal suppository is about 2 to 3 gm.

Tablets and capsules for rectal administration are manufactured using the same pharmaceutically acceptable substance and by the same methods as for formulations for oral administration.

### g. Articles of manufacture

The compounds provided herein may be packaged as articles of manufacture containing packaging material and a label that indicates that the compound is used for treating, ameliorating or preventing a cystic disease. The articles of manufacture provided herein contain packaging materials. Packaging materials for use in packaging pharmaceutical products are well known to those of skill in the art. See, e.g., U.S. Patent Nos. 5,323,907, 5,052,558 and 5,033,352. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. A wide array of formulations of the compounds provided herein are contemplated herein.

### G. EVALUATION OF THE ACTIVITY OF THE COMPOUNDS

Standard physiological, pharmacological and biochemical procedures are available and are known to one of skill in the art to test the efficacy of the compounds in the methods provided herein. Such procedures include but are not limited to *in vitro* cystogenesis assays and *in vivo* efficacy studies using two different mouse models of PKD: jck (slowly progressive) and cpk (agressive). Jck mice develop focal cysts at 3 days of life, by 26 days the cystic disease is established and by day 50 most of the kidney is involved in cystogenesis. An exemplary treatment schedule for jck mice includes daily intraperitoneal (IP) injections of a compound provided herein from day 26 to day 50 or to day 64. At the end of the study mice are sacrificed and the following end points are taken:
1. kidney to body weight ratio
2. Histology: Cyst percentage is measured quantitatively with Metamarph® as a ratio of cystic area to a total section area and used in calculations of cystic volumes (percent of body weight).
3. Renal function: serum blood urea nitrogen (BUN) and creatinine.

Methods for determining these end points are known to one of skill in the art. Exemplary methods are described herein.

Effectiveness of a compound in treating PKD is indicated by reduction in kidney/body weight ratio, reduction in cystic volume and/or reduction in BUN and creatinine.

It is understood that the foregoing detailed description and accompanying examples are merely illustrative, and are not to be taken as limitations upon the scope of the subject matter. Various changes and modifications to the disclosed embodiments will be apparent to those skilled in the art. Such changes and modifications, including without limitation those relating to the chemical structures, substituents, derivatives, intermediates, syntheses, formulations and/or methods of use provided herein, may be made without departing from the spirit and scope thereof. U.S. patents and publications referenced herein are incorporated by reference.

### EXAMPLES

Jck and cpk mouse models of PKD (C57BL/6J jck /+ mice and C57BL/6J cpk/+ mice) were obtained from The Jackson Laboratory (Bar Harbor, ME, USA) and used to establish a breeding colony maintained at Biological Research Models (B.R.M., Worcester, MA, USA) and Genzyme (Framingham, MA, USA). R-Roscovitine, (6-benzylamino-2-[R-1-ethyl-2-hydroxyethylamino]-9-isapropylpurine) was obtained from A.G. Scientific (San Diego, CA). Cystic jck/jck mice were identified by a custom genotyping assay, divided into control and treated groups and injected intraperitoneally (IP) with either vehicle or drug at specified concentrations. In the examples, below, roscovitine refers to R-roscovitine.

### Example 1

Beginning at postnatal day 26 jck/jck mice received either Roscovitine (50 or 150 mg/kg) or vehicle (5% Ethanol, 5% Cremophor in buffered saline solution, pH 7.2) for 3 or 5 weeks. Pulse therapy was performed with 150 mg/kg by IP injection daily starting at day 26 for 3 weeks followed by 2 weeks without treatment or by alternating one week of treatment with one week without treatment for 5 weeks total.

Cpk animals (entire litters from cpk/+ heterozygous breeders) were daily IP injected beginning at postnatal day 7 with either Roscovitine (100 mg/kg) or vehicle for 2 weeks.

Mice were euthanized by CO₂ asphyxiation and kidneys were processed for protein extraction (snap frozen) and for histology examination (fixed in 4% PFA buffered solution, pH 7.2). Blood was collected by cardiac puncture and serum urea nitrogen and creatinine levels were measured using a VetACE analyzer (Alfa Wasserman, West Coldwell, NJ).

### Morphometric analysis of cystogenesis

Paraffin embedded kidneys were cut to obtain four micrometers longitudinal and cross sections. Sections were stained with hematoxylin and eosin (H&E) with a Tissue Tek® 2000 processor (Sakura-Finetek, Torrance, CA). The slides were digitized with an ACIS®) system (Clarient, San Juan Capistrano, CA) and processed with the MetaMorph Imaging Series® software (Molecular Devices Corp, Downingtown, PA). To verify data generated with MetaMorph, kidney sections were also assessed with Scion Image software (Scion Corp, Frederick, MD). The data generated with MetaMorph and Scion software were almost identical with less than 0.5% of variation. The degree of cystogenesis was quantified from both longitudinal and transverse sections for each mouse. Dilations with diameter greater than 5x average normal tubule diameter were counted as cysts. Cystic percentage was measured as a ratio of cystic area to a total section area and used in calculations of cystic volumes (percent of body weight) as previously described by Torres *et al.,* Effective treatment of an orthologous model of autosomal dominant polycystic kidney disease *Nat. Med.* (2004).

As seen from the data presened in Table 2 and Table 2a (for jck mice model), roscovitine was well tolerated with no weight loss, mortality or toxicity. Roscovitine-treated group showed reduction of the kidney to body weight ratio and cystic volumes in a dosedependent manner. BUN (blood urea nitrogen) was reduced in 50 mg/kg Roscovitine treated group and normalized with 150 mg/kg Roscovitine. Roscovitine also inhibited PKD in jck/jck females, where disease is less severe than in males.

In a pulse treatment study where 150 mg/kg of Roscovitine was administered daily to jck/jck males for 3 weeks, followed by 2 weeks without treatment, a long lasting anti-cystic effect was detected with normalized levels of BUN. In a different schedule where the drug was administered daily for one week followed by one week off treatment for a total of 5 weeks, reduction of cystogenesis was detected. Therefore, in certain embodiments, Roscovitine shows long lasting treatment effect after drug withdrawal and is also efficacious with intermittent dosing.

Table 3 provides data for cpk model. As seen from the data, daily treatment of cpk mice with 100 mg/kg Roscovitine from day 7 till day 21 resulted in reduction of PKD.

**Table 2. Anti-cystic effect of Roscovitine in jck mouse model of PKD**

| **No** | | **Gender** | **Dose (mg/kg)** | **Treatment (weeks)** | **No of animals** | **Body weight (g)** | **K/BW ratio (%)** | **BUN (mg/dL)** | **Cystic volume (%BW)** |
|---|---|---|---|---|---|---|---|---|---|
| | **Continuous treatment** | | | | | | | | |
| 1 | Vehide | F | - | 5W | 10 | 20.09 ± 0.86 | 5.71 ± 0.58 | 52.67 ± 12.30 | 1.66 ± 0.54 |
| 2 | Roscovitine | F | 150 | 5W | 12 | 19.79 ± 0.80 | 3.49 | 31.09 ± 6.12* | 0.74 ± 0.24 * |
| | | | | | | | | | ± |
| 3 | Vehicle | M | - | 5W | 17 | 20.75 ± 1.56 | 9.07 ± 0.69 | 79.00 ± 14.47 | 3.63 0.53 * |
| 4 | Roscovitine | M | 150 | 5W | 9 | 21.52 ± 1.78 | 4.17 ± 0.26 * | 26.78 ± 3.31 | 0.98 ± 0.15 |
| 5 | Roscovitine | M | 50 | 5W | 8 | 20.54 ± 1.66 | 6.55 ± 1.06 * | 51.25 ± 10.19 | * 2.25 * 0.66 * |
| | | | | | | | | | |

| | **Pulse treatment** | | | | | | | | |
|---|---|---|---|---|---|---|---|---|---|
| 7 | Vehicle | M | - | 5W (1/1** | 6 | 23.21 ± 0.90 | 7.59 ± 0.96 | 63.67 ± 10.33 | 2.79 ± 0.72 |
| 8 | Roscovitine | M | 150 | 5W (3/2**) | 5 | 22.77 ± 0.39 | 4.71 ± 0.69 * | 27.60 ± 4.32 | * 1.29 ± 0.40 * |
| 9 | Roscovitine | M | 150 | 5W (1/1**) | 9 | 22.14 ± 1.10 | 4.56 ± 0.62 ***** | 43.90 ± 13.08 | 1.28 ± 0.22 * |
| | | | | | | | | | |
| 10 | Wild type (50 d) | F | - | - | 6 | 18.66 ± 1.09 | 1.36 ± 0.10 | 22.83 ± 2.50 | N/A |
| 11 | Wild type (50 d) | M | - | - | 7 | 25.07 ± 0.78 | 1.40 ± 0.10 | 25.57 ± 2.33 | N/A |

| | | | | | | | | | |
|---|---|---|---|---|---|---|---|---|---|
| * indicates P<0.05 Roscovitine treated vs. vehicle treated animals N/A- not applicable ** indicates pulse treatment, for example 5W (1/1) indicates 1 week on roscovitine followed by 1 week off for 5 weeks. | | | | | | | | | |

**Table 2a. Effect of Roscovitine (daily dosing for 5 weeks) on PKD progression in the jck mice (male)**

| **No** | | **Dose (mg/kg)** | **No of animals** | **Body weight (g)** | **K/BW ratio (%)** | **BUN (mg/dL)** | **Cystic volume (%BW)** |
|---|---|---|---|---|---|---|---|
| 1 | Vehicle | - | 10 | 20.95 ± 1.56 | 8.76 ± 0.56 | 77.30 ± 13.10 | 4.06 ± 0.44 |
| 2 | Roscovitine | 150 | 9 | 22.33 ± 1.05 | 4.23 ± 0.53 * | 28.89 ± 7.51 * | 1.10 ± 0.41 * |

| | | | | | | | |
|---|---|---|---|---|---|---|---|
| * indicates P<0.05 Roscovitine treated vs. vehicle treated animals | | | | | | | |

**Table 3. Anti-cystic effect of Roscovitine in cpkmodel of PKD**

| **No** | | **No of animals** | **Body weight (g)** | **KIBW ratio (%)** | **Creatinine (mg/dL)** | **BUN (mg/dL)** | **Cystic volume(%BW)** |
|---|---|---|---|---|---|---|---|
| 1 | Vehicle | 23 | 9.21 ± 0.73 | 25.31 ± 2.13 | 0.46 ± 0.10 | 70.39 ± 16.50 | 19.21 ± 1.62 |
| 2 | Roscovitine | 20 | 9.75 ± 1.00 | 1925 ± 3.17 * | 0.29 ± 0.07 * | 54.58 ± 9.60 * | 14.62 ± 2.16 * |

| | | | | | | | |
|---|---|---|---|---|---|---|---|
| * indicates P<0.05 Roscovitine treated vs. vehicle treated animals | | | | | | | |

### Immunohistochemical and immunofluorescence examination

Normal and ADPKD formalin-fixed paraffin-embedded kidney specimens were obtained from Cytomix LLC (Lexington, MA). Four micrometers kidney sections were treated with proteinase K or boiled in Citra Antigen Retrieval solution (Biogenex, San Ramon, CA) in pressure cooker for unmasking antigens. PCNA staining was performed as described in the manufacturer's protocol for the M.O.M. kit (Vector Laboratories, Burlingame, CA). TUNEL staining was performed according to the manufacturer's protocol for the ApopTag Apoptosis Detection Kit (Chemicon, Temecula, CA). Lectin Dolichos Biflorus Agglutinin (DBA) (Vector Laboratories, Burlingame, CA) was used at the dilution 1:50. Anticalbindin (Sigma-Aldrich, St. Louis, MO) and anti-Tamm-Horsfall glycoprotein (US Biological, Swampscott, MA) antibodies were used as recommended by manufacturers. Secondary antibody (Sigma-Aldrich) conjugated to Cy3 or FITC were used at the dilution 1:100. Staining was visualized on an Olympus IX70 microscope with 20x objective (Olympus-America, Melville, NY). Images were captured with QED Camera Plug-In imaging system (QED imaging, Pittsburgh, PA).

High proliferation rates for human ADPKD cyst-lining epithelia were confirmed using PCNA immunostaining. Likewise, high number of PCNA-positive cells were seen in jck cysts. In Roscovitine treated jck kidney, quatitative reduction in PCNA-positive cells were observed. Roscovitine treatment also reduced the number of TUNEL -positive cells.

### Protein purification and Western blot analysis

Kidneys were homogenized in RIPA buffer (10 mM HEPES, pH 8.0, 100 mM NaCl, 1% Triton X-100, 0.1% SDS, 0.5% sodium deoxycholate, 1 mM DTT, 1 mM EDTA, 1 mM NaF, 1 mM Na3VO4) with CompleteTM protease inhibitor cocktail (Roche Diagnostic GmbH, Mannheim, Germany). 30 µg of protein samples were separated in 4-12% SDS/acrylamide gels (Invitrogen, Carlsbad, CA). Gels were soaked in transfer buffer (192 mM glycine, 25 mM Tris, 10% methanol, pH 8.3) and transferred to Immobilone-P membranes (Millipore Corporation, Bedford, MA). Membranes were rehydrated with methanol and incubated in blocking buffer (5% powdered milk or 3% BSA in PBS) for 30 min. Membranes were incubated overnight at 4°C with primary antibodies against RNApoIII-p, p35/p25 (Abcam, Cambridge, MA); Rb-p, cycD1, cycD1-p, ERK2, ERK1/2-p (Cell Signaling, Danvers, MA); Rb2, RBBP, cycD3, caspase-2, caspase-3, ApaF1, Bcl-2, Bcl-XL, BAD (BD Biosciences, San Jose, CA); CDK7, CDK9, PCNA, GAPDH (US Biologicals, Swampscott, MA); cycD2 (BioSource International, Camarillo, CA), washed in TBST and incubated with HRP-labeled secondary antibodies as recommended by the manufacturer. Immunoreactive proteins were detected using enhanced chemi-luminescence (Amersham Pharmacia Biotech, Little Chalfont Buckinghamshire, England).

Data obtained indicated that Rb-p form was increased in cystic kidneys. Roscovitine treatment resulted in dephosphorylation of Rb3 suggesting effective G1/S cell cycle block. Also, Roscovitine treatment normalized the levels of Rb associated proteins Rb2 and RBB2. Although cyclin D1 level was not substantially increased in cystic kidneys, its phosphorylation (Thr-286) was decreased. Roscovitine normalized phosphorylation level of cyclin D1. The levels of Cyclin D2 and D3 expression were increased in cystic kidneys and downregulated by Roscovitine. Roscovitine also affected signaling from Ras-Raf to MEK-ERKs, known to regulate cyclin D1 by decreasing the level of ERK 1/2 activation. Roscovitine also inhibits CDK7 and CDK9 that regulate transcription by phosphorylating the C-terminal domain of RNA polymerase II (RNA pol II). Moderately increased levels of CDK7 and CDK9 detected in cystic kidneys resulted in greatly increased phosphorylation of RNA pol II. In contrast, Roscovitine treated samples were almost indistinguishable from WT controls showing reduced levels of RNA pol II phosphorylation. Thus, Roscovitine attenuates cystogenesis through cell cycle arrest and transcriptional inhibition.

### Example 2: In vitro inhibition of cystogenesis

Standard assay of MDCK cystogenesis in vitro was used as previously described. (*See,* Bukanov, N. O. et al. Functional polycystin-1 expression is developmentally regulated during epithelial morphogenesis in vitro: downregulation and loss of membrane localization during cystogenesis. Hum. Molec. Genetics 11, 923-936 (2002) and Li, H., et al. The relationship between cell proliferation, Cl- secretion, and renal cyst growth: a study using CFTR inhibitors. Kidney Int 66, 1926-38 (2004)). Briefly, MDCK kidney epithelial cells (ATCC, Rockville, Maryland) were grown in MEM/10%FBS. MDCK cysts were cultured in 3D collagen I gel (BD Biosciences, Bedford, MA) in 96 well plates for four days until cystic lumens were fully formed. Increasing concentrations of R-Roscovitine, S-Roscovitine and N6-methyl-(R)-Roscovitine were added to cystic cultures and incubated for 96 hours. Each concentration was assayed in quadruplicates. Images of cysts were acquired on Zeiss Axiovert 25 inverted microscope with a 20x objective. Inhibition of cystic growth was quantified using Alamar Blue assay (BioSource, Camarillo, CA). Fluorescence was measured with microplate reader (Molecular Devices, Spectra Max Gemini, Sunnyvale, CA).

R-Roscovitine (R-Rosc) and S-Roscovitine (S-Rosc) specifically inhibited cystic growth with IC₅₀ of about 20 µM and about 30 µM respectively, while N6-methyl-(R)-Roscovitine (N6-met-R-Rosc) did not show substantial inhibition (IC₅₀ of 190 µM),

Since modifications will be apparent to those of skill in this art, it is intended that this invention be limited only by the scope of the appended claims.
The invention also relates to the following items:
1. A method for treating or ameliorating one or more symptoms of a cystic disease, comprising administering a therapeutically effective amount of a compound of formula I 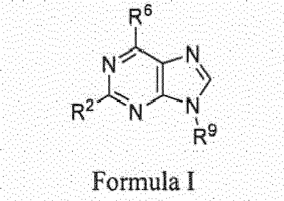 or a pharmaceutically acceptable derivative thereof, wherein
   R² and R⁶ are each independently selected from halo, pseudohalo, alkyl, alkenyl, alkynyl, aryl, heteraaryl, cycloalkyl, heterocyclyl, R-NH-, R-NH-NH-, NH2-R¹-NH- and R-NH-R¹-NH-;
   R⁹ is hydrogen, alkyl, alkenyl, alkynyl, aryl, heteroaryl, cycloalkyl, heterocyclyl, R-NH-, R-NH-NH-, NH₂-R¹-NH- or R-NH-R¹-NH-;
   R is H, alkyl, alkenyl, alkynyl, aryl, heteroaryl, cycloalkyl or heterocyclyl;
   R¹ is alkynyl, alkenylene, alkynylene, cycloalkynyl, heterocyclylene, arylene, or heteroarylene; and
   R, R¹, R², R⁶, and R⁹ groups are optionally substituted with one, two, three or four Q groups, each independently selected from -OH, -COOH, halo, pseudohalo, amino, alkyl, alkenyl, alkynyl, cycloalkyl, heterocyclyl, aryl and heteroaryl.
2. The method of item 1, wherein R is alkyl, aralkyl, hydroxyalkyl, aryl, cycloalkyl or heterocyclyl.
3. The method of item 1 or 2, wherein R' is alkynyl, arylene or cycloalkynyl.
4. The method of any of items 1-3, wherein Q is -OH, halo, pseudohalo, amino or alkyl.
5. The method of any of items 1-4, wherein R² is hydroxyalkylamino.
6. The method of any of items 1-5, wherein R² is (1-ethyl-2-hydroxy)ethylamino.
7. The method of any of items 1-4, wherein R⁶ is aralkylamino.
8. The method of any of items 1-4, wherein R⁶ is benzylamino.
9. The method of any of items 1-8, wherein R⁹ is isopropyl.
10. The method of any of items 1-9, wherein the compound is 6-benzylamino-2-[(1-ethyl-2-hydroxy)ethylamino]-9-isopropylpurine.
11. The method of any of items 1-10, wherein the compound is 6-benzylamino-2-(*R*)-[(1-ethyl-2-hydroxy)ethylamino]-9-isopropylpurine.
12. The method of item 1, wherein the compound is selected from 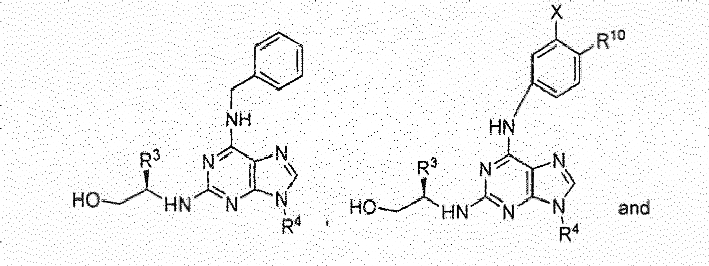 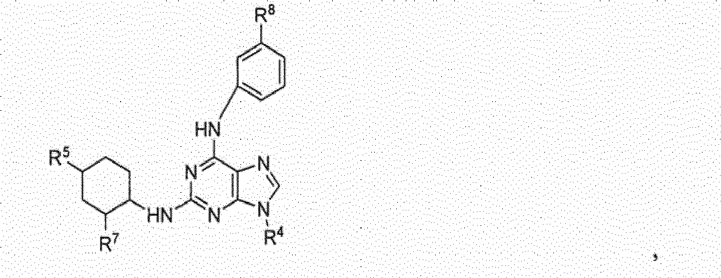 wherein
   R³ and R⁴ are each independently H, methyl, ethyl or isopropyl;
   R¹⁰ is H or COOH;
   R⁸ is H, halo or pseudohalo;
   R⁵ is H or OH;
   R⁷ is H or NH₂ and X is halo.
13. The method of items 1 or 12, wherein the compound is selected from 2-(2-hydroxyethylamino)-6-benzylamino-9-methylpurine, 2-(2'-Hydroxyethylamino)-6-benzylamino-9-isopropylpurine, (2*R*)-2-[[6-[(3-chlorophenyl)amino]-9-propan-2-ylpurin-2-yl]amino]-3-methylbutan-1-ol and (2*R*)-2-[[6-[(3-chloro-4-carboxyphenyl)amino]-9-(1-methylethyl)-9*H*-purin-2-yl]amino]-3-methyl-1-butanol.
14. The method of items 1 or 12, wherein the compound is 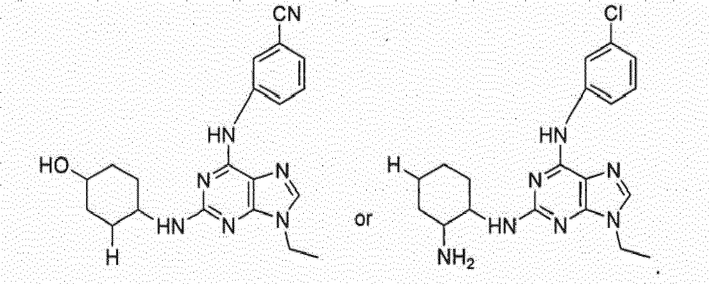
15. The method of item 1, wherein the compound is selected from 2-(1-D,L-hydroxymethylpropylamino)-6-benzylamino-9-isopropylpurine, non-crystalline 6-benzylamino-2-[(2R)-2-hydroxymethyl-pyrrolidin-1-yl]-9-isopropyl-(9H)-purine, 2-(R)-[6-benzylamino-9-isopropyl-(9H)-purin-2-yl]-amino-2-phenylethanol, 2-(R,S)-[6-benzylamino-9-isopropyl-(9H)-purin-2-yl]-amino-pentanol, 2-(R)-[6-benzylamino-9-isopropyl-(9H)-purin-2-yl]-amino-propanol, 2-(S)-[6-benzylamino-9-isopropyl-(9H)-purin-2-yl]-amino-propanol, 2-(R)-(-)-[6-(3-iodo)-benzylamino-9-isopropyl-(9H)-purin-2-yl]-N-pyrrolidine-methanol and 2-(R)-(-)-[6-benzylamino-9-cyclopentyl-(9H)-purin-2-yl]-N-pyrrolidine-methanol.
16. The method of any of items 1-15, wherein the compound is administered in a single dosage form.
17. The method of any of items 1-15, wherein the compound is administered in a pulse dosing schedule.
18. The method of item 17, wherein the pulse dosing schedule comprises administering the compound to a subject for three consecutive weeks followed by a period of three drug-free weeks.
19. The method of item 17, wherein the pulse dosing schedule comprises administering the compound to a subject for two consecutive weeks followed by a period of two drug-free weeks.
20. The method of item 17, wherein the pulse dosing schedule comprises administering the compound to a subject for 10 consecutive days followed by a period of 10 drug-free days.
21. The method of item 17, wherein the pulse dosing schedule comprises
   administering the compound to a subject for one week followed by a period of one drug-free week.
22. The method of any of items 1-21, wherein the cystic disease is selected from acquired renal cystic disease, dialysis-associated cystic disease, autosomal dominant polycystic kidney disease, autosomal recessive polycystic kidney disease, congenital multicystic kidney, multicystic dysplastic kidney, end-stage renal disease, medullary sponge kidney, MSK, nephronophthisis-medullary cystic kidney disease complex, nephronophthisis-uremic medullary cystic disease complex, juvenile nephronophthisis, medullary cystic disease, renal cell carcinoma, tuberous sclerosis and von Hippel-Lindau syndrome.
23. The method of any of items 1-21, wherein the cystic disease is a polycystic kidney disease.
24. The method of any of items 1-23, wherein the compound is administered orally.
25. An article of manufacture comprising packaging material and a compound of Formula I, contained within the packaging material, wherein the packaging material includes a label that indicates that the compound is used for treating a cystic disease, wherein the compound has Formula I 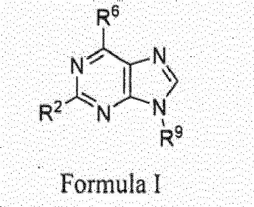 or a pharmaceutically acceptable derivative thereof, wherein
   R² and R⁶ are each independently selected from halo, pseudohalo, alkyl, alkenyl, alkynyl, aryl, heteroaryl, cycloalkyl, heterocyclyl, R-NH-, R-NH-NH-, NH₂-R¹-NH- and R-N H-R¹-NH-;
   R⁹ is hydrogen, alkyl, alkenyl, alkynyl, aryl, heteroaryl, cycloalkyl, heterocyclyl, R-NH-, R-NH-NH-, NH₂-R¹-NH- or R-NH-R¹-NH-;
   R is H, alkyl, alkenyl, alkynyl, aryl, heteroaryl, cycloalkyl or heterocyclyl;
   R' is alkynyl, alkenylene, alkynylene, cycloalkynyl, heterocyclylene, arylene, or heteroarylene; and
   the R, R¹, R², R⁶, and R⁹ groups are optionally substituted with one, two, three or four Q groups, each independently selected from -OH, -COOH, halo, pseudohalo, amino, alkyl, alkenyl, alkynyl, cycloalkyl, heterocyclyl, aryl and heteroaryl.

## Claims

1. A compound of formula I 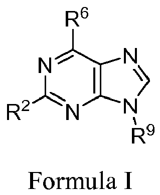 or a pharmaceutically acceptable derivative thereof for use in a method for treating or ameliorating one or more symptoms of a cystic disease, wherein
R² and R⁶ are each independently selected from halo, cyano, thiocyanate, selenocyanate, trifluoromethoxy, azide, alkyl, alkenyl, alkynyl, aryl, heteroaryl, cycloalkyl, heterocyclyl, R-NH-, R-NH-NH-, NH₂-R¹-NH- and R-NH-R¹-NH-;
R⁹ is hydrogen, alkyl, alkenyl, alkenyl, aryl, heteroaryl, cycloalkyl, heterocyclyl, R-NH-, R-NH-NH-, NH₂-R¹-NH- or R-NH-R¹-NH-;
R is H, alkyl, alkenyl, alkenyl, aryl, heteroaryl, cycloalkyl or heterocyclyl;
R¹ is alkenyl, alkenylene, alkenylene, cycloalkynyl, heterocyclylene, arylene, or heteroarylene; and
R, R¹, R², R⁶, and R⁹ groups are optionally substituted with one, two, three or four Q groups, each independently selected from -OH, -COOH, halo, cyano, thiocyanate, selenocyanate, trifluoromethoxy, azide, amino, alkyl, alkenyl, alkynyl, cycloalkyl, heterocyclyl, aryl and heteroaryl.

2. The compound for use of claim 1, wherein R is alkyl, aralkyl, hydroxyalkyl, aryl, cycloalkyl or heterocyclyl.

3. The compound for use of claim 1 or 2, wherein R¹ is alkynyl, arylene or cycloakynyl.

4. The compound for use of any of claims 1-3, wherein Q is -OH, halo, pseudohalo, amino or alkyl.

5. The compound for use of any of claims 1-4, wherein R² is hydroxyalkylamino, optionally wherein R² is (1-ethyl-2-hydroxy)ethylamino.

6. The compound for use of any of claims 1-4, wherein R⁶ is aralkylamino, optionally wherein R⁶ is benzylamino.

7. The compound for use of any of claims 1-6, wherein R⁹ is isopropyl.

8. The compound for use of any of claims 1-7, wherein the compound is 6-benzylamino-2-[(1-ethyl-2-hydroxy)ethylamino]-9-isopropylpurine, optionally wherein the compound is 6-benzylamino-2-(R)-[(1-ethyl-2-hydroxy)ethylamino]-9-isopropylpurine.

9. The compound for use of claim 1, wherein the compound is selected from 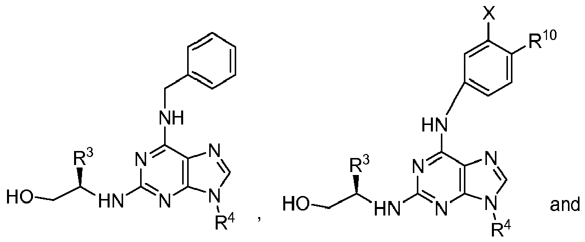 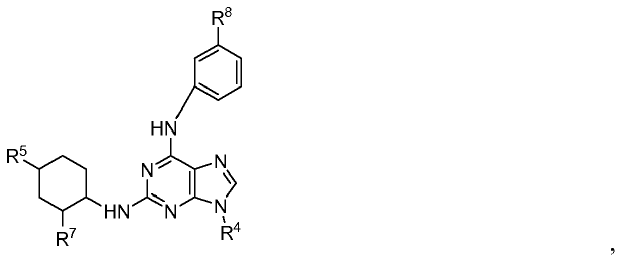 wherein
R³ and R⁴ are each independently H, methyl, ethyl or isopropyl;
R¹⁰ is H or COOH;
R⁸ is H, halo or cyano, thiocyanate, selenocyanate, trifluoromethoxy, azide;
R⁵ is H or OH;
R⁷ is H or NH₂ and X is halo; or
wherein the compound is selected from 2-(2-hydroxyethylamino)-6-benzylamino-9-methylpurine, 2-(2'-Hydroxyethylamino)-6-benzylamino-9-isopropylpurine, (2*R*)-2-[[6-[(3-chlorophenyl)amino]-9-propan-2-ylpurin-2-yl]amino]-3-methylbutan-1-ol and (2*R*)-2-[[6-[(3-chloro-4-carboxyphenyl)amino]-9-(1-methylethyl)-9*H*-purin-2-yl]amino]-3-methyl-1-butanol; or
wherein the compound is 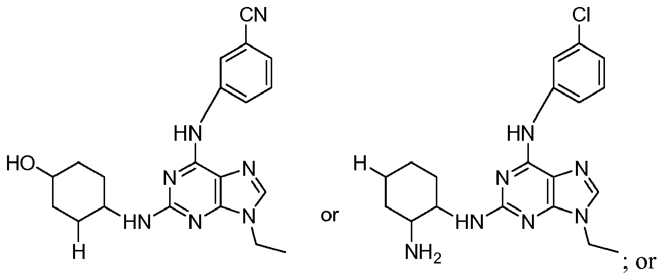 wherein the compound is selected from 2-(1-D,L-hydroxymethylpropylamino)-6-benzylamino-9-isopropylpurine, non-crystalline 6-benzylamino-2-[(2R)-2-hydroxymethyl-pyrrolidin-1-yl]-9-isopropyl-(9H)-purine, 2-(R)-[6-benzylamino-9-isopropyl-(9H)-purin-2-yl]-amino-2-phenylethanol, 2-(R,S)-[6-benzylamino-9-isopropyl-(9H)-purin-2-yl]-amino-pentanol, 2-(R)-[6-benzylamino-9-isopropyl-(9H)-purin-2-yl]-amino-propanol, 2-(S)-[6-benzylamino-9-isopropyl-(9H)-purin-2-yl]-amino-propanol, 2-(R)-(-)-[6-(3-iodo)-benzylamino-9-isopropyl-(9H)-purin-2-yl]-N-pyrrolidine-methanol and 2-(R)-(-)-[6-benzylamino-9-cyclopentyl-(9H)-purin-2-yl]-N-pyrrolidine-methanol.

10. The compound for use of any of claims 1-9, wherein the compound is administered in a single dosage form.

11. The compound for use of any of claims 1-9,
wherein the compound is administered in a pulse dosing schedule; optionally
wherein the pulse dosing schedule comprises administering the compound to a subject for three consecutive weeks followed by a period of three drug-free weeks, or
wherein the pulse dosing schedule comprises administering the compound to a subject for two consecutive weeks followed by a period of two drug-free weeks, or
wherein the pulse dosing schedule comprises administering the compound to a subject for 10 consecutive days followed by a period of 10 drug-free days, or
wherein the pulse dosing schedule comprises administering the compound to a subject for one week followed by a period of one drug-free week.

12. The compound for use of any of claims 1-11, wherein the cystic disease is selected from acquired renal cystic disease, dialysis-associated cystic disease, autosomal dominant polycystic kidney disease, autosomal recessive polycystic kidney disease, congenital multicystic kidney, multicystic dysplastic kidney, end-stage renal disease, medullary sponge kidney, MSK, nephronophthisis-medullary cystic kidney disease complex, nephronophthisis-uremic medullary cystic disease complex, juvenile nephronophthisis, medullary cystic disease, renal cell carcinoma, tuberous sclerosis and von Hippel-Lindau syndrome.

13. The compound for use of any of claims 1-11, wherein the cystic disease is a polycystic kidney disease.

14. The compound for use of any of claims 1-13, wherein the compound is administered orally.

15. An article of manufacture comprising packaging material and a compound of Formula I, contained within the packaging material, wherein the packaging material includes a label that indicates that the compound is used for treating a cystic disease, wherein the compound has Formula I 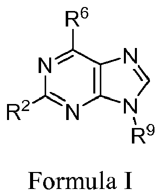 or a pharmaceutically acceptable derivative thereof, wherein
R² and R⁶ are each independently selected from halo, pseudohalo, alkyl, alkenyl, alkynyl, aryl, heteroaryl, cycloalkyl, heterocyclyl, R-NH-, R-NH-NH-, NH₂-R¹-NH- and R-NH-R¹-NH-;
R⁹ is hydrogen, alkyl, alkenyl, alkenyl, aryl, heteroaryl, cycloalkyl, heterocyclyl, R-NH-, R-NH-NH-, NH₂-R¹-NH- or R-NH-R¹-NH-;
R is H, alkyl, alkenyl, alkenyl, aryl, heteroaryl, cycloalkyl or heterocyclyl;
R¹ is alkenyl, alkenylene, alkenylene, cycloalkynyl, heterocyclylene, arylene, or heteroarylene; and
the R, R¹, R², R⁶, and R⁹ groups are optionally substituted with one, two, three or four Q groups, each independently selected from -OH, -COOH, halo, cyano, thiocyanate, selenocyanate, trifluoromethoxy, azide, amino, alkyl, alkenyl, alkynyl, cycloalkyl, heterocyclyl, aryl and heteroaryl.
